# EUROPÄISCHE PATENTSCHRIFT

(11) **EP 0 680 464 B1**
(45) Veröffentlichungstag und Bekanntmachung des Hinweises auf die Patenterteilung: **09.04.1997**
(21) Anmeldenummer: 94904643.7
(22) Anmeldetag: 08.01.1994
(51) Int. Cl.: C07C 229/16, A61K 49/00

(54) **DERIVATISIERTE DTPA-KOMPLEXE, DIESE VERBINDUNGEN ENTHALTENDE PHARMAZEUTISCHE MITTEL, IHRE VERWENDUNG ALS NMR- UND RÖNTGENKONTRASTMITTEL UND VERFAHREN ZU DEREN HERSTELLUNG**
DERIVATED DTPA COMPLEXES, PHARMACEUTICALS CONTAINING THESE COMPOUNDS, THEIR USE AS NMR- AND RADIOGRAPHIC CONTRASTING AGENTS AND PROCESS FOR PRODUCING THE SAME
COMPLEXES DERIVES DE DTPA, AGENTS PHARMACEUTIQUES RENFERMANT CES COMPOSES, LEUR UTILISATION COMME PRODUITS DE CONTRASTE POUR RMN ET RADIOGRAPHIE, ET LEUR PROCEDE DE FABRICATION

(30) Priorität: 25.01.1993 DE 4302287
(43) Veröffentlichungstag der Anmeldung: 08.11.1995
(73) Patentinhaber: SCHERING AKTIENGESELLSCHAFT, 13353 Berlin (DE)
(72) Erfinder: SCHMITT-WILLICH, Heribert, D-12161 Berlin (DE); PLATZEK, Johannes, D-14195 Berlin (DE); GRIES, Heinz, D-10717 Berlin (DE); RADÜCHEL, Bernd, D-13465 Berlin (DE); PETROV, Orlin, D-14199 Berlin (DE); MÜHLER, Andreas, D-10319 Berlin (DE); FRENZEL, Thomas, D-12247 Berlin (DE); VOGLER, Hubert, D-10629 Berlin (DE); BAUER, Hans, D-12107 Berlin (DE); NICKISCH, Klaus, D-12307 Berlin (DE); HILSCHER, Jean-Claude, D-14195 Berlin (DE)
(86) Internationale Anmeldenummer: EP9400033
(87) Internationale Veröffentlichungsnummer: WO9417029

(56) Entgegenhaltungen:
- EP-A- 0 071 564
- EP-A- 0 258 616
- EP-A- 0 299 795
- EP-A- 0 405 704
- WO-A-91/14459
- DE-B- 1 155 122

## Beschreibung

Die Erfindung betrifft den in den Patentansprüchen gekennzeichneten Gegenstand, das heißt neue Komplexe und Komplexsalze, diese Verbindungen enthaltende Mittel, ihre Verwendung in der Diagnostik und Strahlentherapie sowie Verfahren zur Herstellung dieser Verbindungen und Mittel.

Metallkomplexe sind schon zu Beginn der 50er Jahre als Kontrastmittel für die Radiologie in Betracht gezogen worden. Die damals eingesetzten Verbindungen waren aber derart toxisch, daß eine Verwendung beim Menschen nicht in Betracht kam. Es war daher durchaus überraschend, daß sich bestimmte Komplexsalze als ausreichend verträglich erwiesen haben, so daß eine routinemäßige Anwendung am Menschen für diagnostische Zwecke in Erwägung gezogen werden konnte. Als erster Vertreter dieser Substanzklasse hat sich das in der europäischen Patentanmeldung mit der Publikationsnummer 0 071 564 beschriebene Dimegluminsalz des GdDPTA (Gadolinium-III-Komplex der Diethylentriaminpentaessigsäure; Magnevist®) als Kontrastmittel für die Kernspintomographie sehr gut bewährt.

Dieses Kontrastmittel ist besonders gut für die Diagnose pathologischer Bereiche (z.B. Entzündungen, Tumore, Infarkte etc.) geeignet. Nach intravenöser Injektion verteilt sich die Verbindung extrazellulär und wird durch glomeruläre Filtration über die Nieren eliminiert. Eine Passage intakter Zellmembranen und eine extrarenale Ausscheidung werden praktisch nicht beobachtet.

Auch die in den Patentanmeldungen EP 0 305 320 und EP 0 299 795 beschriebenen Metallkomplexe verteilen sich extrazellulär und werden nahezu ausschließlich renal ausgeschieden. Für den Patienten mit eingeschränkter Nierenfunktion sind die genannten Kontrastmittel daherweniger geeignet, da die Ausscheidung bei diesen nur langsam erfolgt, sodaß die vollständige Entfernung des Kontrastmittels aus dem Organismus oft nur mit Hilfe eines Dialysegerätes gelingt.

Aufgrund ihres pharmakokinetischen Verhaltens sind diese Kontrastmittel auch als Diagnostika für den hepatobiliären Bereich nur begrenzt geeignet. Daher besteht ein Bedarf an Kontrastmitteln, die von den Leberzellen aufgenommen werden und dadurch eine bessere Unterscheidung von gesundem Parenchym und tumorösem Gewebe ermöglichen.

Für diesen Anwendungsbereich geeignete Kontrastmittel werden in der EP 0 186 616 beschrieben. Diese Mittel auf der Basis von Dextran-gecoateten, magnetischen Eisenoxid-Partikeln sind jedoch mit dem Nachteil behaftet, daß sie das Eisendepot des Körpers unnötig belasten. Darüber hinaus können diese Verbindungen - wie alle hochmolekularen Verbindungen - bei einer unsachgemäßen Injektion (d.h. bei Verfehlung der Vene) beim Patienten eine unerwünschte, langanhaltende Hämatombildung hervorrufen. Diese Gefahr besteht bei den sich schneller verteilenden niedermolekularen Verbindungen hingegen nicht.

Die EP 0 405 704 beschreibt Metallkomplexe von mit lipophilen Gruppen substituierten DTPA-Derivaten. Diese Verbindungen zeigen prinzipiell das gewünschte Ausscheidungsverhalten, d.h. neben der renalen Ausscheidung wird ein gewisser Anteil des Kontrastmittels auch mit dem Faeces ausgeschieden, jedoch ist insbesondere für eine Anwendung bei niereninsufficienten Patenten noch eine Verbesserung des Ausscheidungsverhälnisses extrarenal/renal wünschenswert.

Auch gilt es, die Relaxivität die als Maß für den bildgebenden Effekt herangezogen werden kann, weiter zu verbessern, um so die für eine diagnostische Aussage notwendige Dosis weiter zu verringern.

Der Erfindung liegt somit die Aufgabe zugrunde, Verbindungen und Mittel bereitzustellen, die die geschilderten Nachteile des Standes der Technik überwinden, d.h. Verbindungen (Mittel) zu finden die in erster Linie extrarenal bzw. hepatobiliär ausgeschieden werden und deren diagnostische Wirksamkeit weiter verbessert ist, sowie ein Verfahren zu deren Herstellung zu schaffen.

Es wurde nun gefunden, daß diese Aufgabe überraschend gelöst wird durch Komplexverbindungen und diese enthaltende Mittel, bestehend aus mindestens einem Metallion eines Elementes der Ordnungszahlen 21-29, 42, 44 oder 58-83 und einem Komplexbildner der allgemeinen Formel I 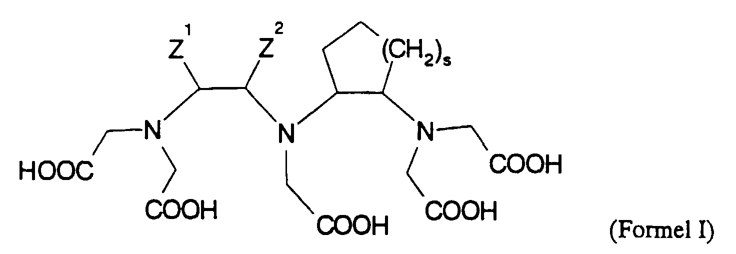 worin
Z¹ und Z² unabhängig voneinander für ein Wasserstoffatom, den Rest -(CH₂)ₘ-(C₆H₄)_{q}-(O)ₖ-(CH₂)ₙ-(C₆H₄)ₗ-(O)ᵣ-R oder den Rest -(CH₂)ₘ-(C₆H₁₀)_{q}-(O)ₖ-(CH₂)ₙ-(C₆H₁₀)ₗ-(O)ᵣ-R stehen,
worin
m und n die Ziffern 0-5
q, k, l und r die Ziffern 0 oder 1 und
s die Ziffern 1 oder 2 bedeuten,
R ein Wasserstoffatom, einen gegebenenfalls OR¹-substituierten geradkettigen oder verzweigten C₁-C₆-Alkylrest oder eine CH₂-COOR¹-Gruppe,
   worin R¹ ein Wasserstoffatom, ein C₁-C₆-Alkylrest oder eine Benzylgruppe bedeutet,
   unter den Maßgaben, daß jeweils einer der Substituenten Z¹ oder Z² für ein Wasserstoffatom steht und der andere nicht für Wasserstoff steht, und daß eine direkte Sauerstoff-Sauerstoff Bindung nicht zugelassen ist,
wobei freie Carbonsäuregruppen, das heißt Carbonsäuregruppen, die nicht zur Komplexierung des Metallions der genannten Ordnungszahlen benötigt werden, gewünschtenfalls teilweise oder vollständig als Salz einer Aminosäure oder eines Aminosäureamids oder als Salz einer anorganischen oder organischen Base vorliegen.

Die erfindungsgemäßen Verbindungen zeigen in überraschend hohem Maße die gewünschte Eigenschaft, d.h. sie werden überwiegend hepatobiliär ausgeschieden wodurch eine Darstellung der Leber, Galle, Gallenblase und Gallenwege möglich wird. Sie zeigen darüber hinaus überraschenderweise im allgemeinen eine höhere Relaxivität als die strukturell ähnlichen Verbindungen der EP 0 405 704 und EP 0 258 616.

Durch die Bereitstellung der erfindungsgemäßen Komplexverbindungen können somit insbesondere niereninsuffiziente Patienten NMR- und Röntgen-diagnostisch untersucht werden.

Die praktische Anwendung der neuen Komplexe wird durch deren hohe chemische Stabilität erleichtert.

Falls das erfindungsgemäße Mittel zur Anwendung in der NMR-Diagnostik bestimmt ist, muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(II)-, Eisen(II)-, Nickel(II)-, Kupfer(II)-, Praseodym(III)-, Neodym(III)-, Samarium(III)-, und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind. Dies sind beispielsweise das Lanthan(III)-ion und die oben genannten Ionen der Lanthanidenreihe.

Die für m und n stehenden Ziffern sind bevorzugt 0-2.

Als Alkylsubstituenten R und R¹ kommen geradkettige, verzweigte oder cyclische Kohlenwasserstoffe mit bis zu 6 Kohlenstoffatomen, die im Falle von R gegebenenfalls durch eine oder mehrere, bevorzugt 1 bis 3 Hydroxy- oder C₁-C₆-, bevorzugt C₁-C₄-Alkoxygruppen substituiert sind, infrage.

Als gegebenenfalls substituierte Alkylgruppen seien beispielsweise die Methyl-, Hydroxymethyl-, Ethyl-, 2-Hydroxyethyl-, 2-Hydroxy-1-(hydroxymethyl)-ethyl-, 1-(Hydroxymethyl)-ethyl-, Propyl-, Isopropyl-, 2- und 3-Hydroxypropyl-, 2.3-Dihydroxypropyl-, n-, sek.- und tert.-Butyl-, 2-, 3-, und 4-Hydroxybutyl-, 2- und 3-Hydroxyisobutyl, Pentyl-, 2-,3- und 4-Hydroxy-2-methylbutyl-, 2.3.4-Trihydroxybutyl-, 1.2.4-Trihydroxybutyl-, Cyclopentyl-, Cyclohexyl-, 2.3.4.5.6.-Pentahydroxyhexylgruppe sowie - im Falle der Hydroxyalkylgruppen, deren C₁-C₆-bevorzugt C₁-C₄-Alkylderivate, genannt.

Bevorzugte Substituenten Z¹ bzw. Z² der erfindungsgemäßen Verbindungen sind der -CH₂-CH(CH₃)₂-, -(CH₂)₄-CH₃-, -CH₂-C₆H₅-, -CH₂-C₆H₄OH-, -CH₂-C₆H₄-OCH₃-,
-CH₂-C₆H₄-OC₂H₅-, -CH₂-C₆H₄-OC₄H₉-, -CH₂-C₆H₄-OCH₂-COOH, -CH₂-C₆H₁₀-OC₂H₅- und der -CH₂-C₆H₄-O-CH₂-C₆H₄-OCH₃-Rest.

Wenn nicht alle aciden Wasserstoffatome durch das Zentralion substituiert sind, können gewünschtenfalls ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Magnesiumion und insbesondere das Calciumion und das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins. Geeignete Kationen von Aminosäureamiden sind beispielsweise die des Lysinmethylamids, Glycinethylamids und des Serinmethylamids.

Die Herstellung der erfindungsgemäßen Komplexverbindungen erfolgt in an sich bekannter Weise, z.B. indem man ein Triamin der allgemeinen Formel II 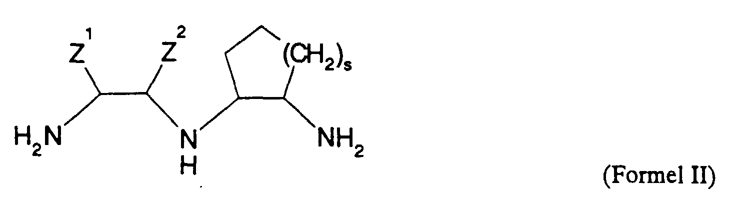 worin Z¹, Z² und s die angegebenen Bedeutungen haben
gegebenenfalls in Gegenwart einer Base, mit einer Verbindung der allgemeinen Formel III

Y-CH₂-COOR² (Formel III),

worin
Y eine Fluchtgruppe und
R² eine Säureschutzgruppe oder ein Wasserstoffatom bedeuten, in einem polaren Lösungsmittel bei -10°C bis 170°C innerhalb von 1-100 Stunden umsetzt, gewünschtenfalls Schutzgruppen abspaltet und den so erhaltenen Komplexbildner der Formel I anschließend mit einem Metallion komplexiert.

Für die Fluchtgruppe Y kann jede dem Fachmann geläufige Abgangsgruppe stehen. Beispielsweise seien Acetat, Brosylat, Mesylat, Nosylat, Tosylat, Trifluoracetat, Trifluorsulfonat, Chlor, Brom oder Jod genannt. Bevorzugte Fluchtgruppen sind Chlor und Brom, besonders bevorzugt ist Brom.

Als Säureschutzgruppen R² kommen niedere Alkyl-, Aryl- und Aralkylgruppen, beispielsweise die Methyl-, Ethyl,- Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Schutzgruppen R² erfolgt nach den dem Fachmann bekannten Verfahren [z.B. E. Wünsch, Methoden der Org. Chemie (Houben-Weyl), Bd. XV/1, 4. Aufl. 1974, S 315 ff] beispielsweise durch Hydrolyse, Hydrogenolyse oder alkalische Verseifung der Ester mit Alkali in wäßrig-alkoholischer Lösung bei Temperaturen von 0 bis 80°C. Zur Abspaltung der für die vorliegenden Reaktionen besonders vorteilhaften t-Butylester können auch organische oder anorganische Säuren verwendet werden: Die in einem geeigneten wasserfreien organischen Lösungsmittel gelöste Esterverbindung, vorzugsweise jedoch die gepulverte Trockensubstanz, wird entweder mit Halogenwasserstoff-Lösung in Eisessig, mit Trifluoressigsäure oder auch Bortrifluoriddiethyletherat in Eisessig versetzt und bei Temperaturen von -10°C bis 60°C, vorzugsweise bei Raumtemperatur, abgespalten.

Verbindungen der allgemeinen Formel I, die in Z¹ bzw. Z² einen Alkoxyphenylrest enthalten, können auch erhalten werden, indem man in an sich bekannter Weise (siehe EP 0 405 704) Verbindungen der allgemeinen Formel IV 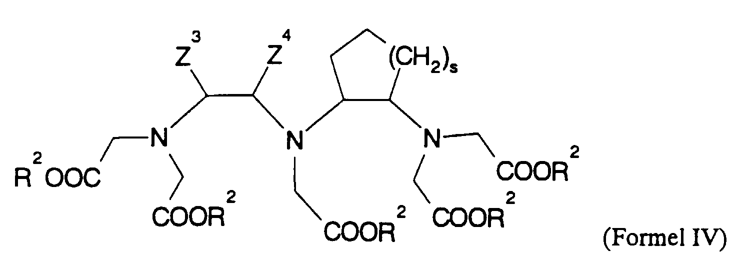 worin
R² und s die angegebenen Bedeutungen haben und
Z³ und Z⁴ jeweils für ein Wasserstoffatom oder den Rest -(CH₂)ₘ-(C₆H₄)_{q}-OH stehen,
mit der Maßgabe, daß einer Substituenten Z³ und Z⁴ für ein Wasserstoffatom und der andere für den angegebenen Rest steht,
in eine Verbindung mit den für Z¹ und Z² angegebenen Resten umwandelt, die Säureschutzgruppen R² in der zuvor beschriebenen Weise abspaltet, die so erhaltenen Komplexbildner der allgemeinen Formel I mit mindestens einem Metalloxid oder Metallsalz eines Elements der Ordnungszahlen 21-29, 42, 44 oder 57-83 umsetzt und anschließend gewünschtenfalls vorhandene acide Wasserstoffatome durch Kationen anorganischer und/oder organischer Basen, Aminosäuren oder Aminosäureamide substituiert.
Als Säureschutzgruppen R² kommen die zuvor genannten Gruppen infrage.

Verbindungen der Formel I, die in Z¹ bzw. Z² einen Cyclohexylrest enthalten, können aus den entsprechenden, in Z³ bzw. Z⁴ einen Benzolring tragenden Verbindungen der allgemeinen Formel IV durch Reduktion z.B. mit Wasserstoff an Rhodium-Kontakten, erhalten werden.

Die Triamine der allgemeinen Formel II können z.B. durch Umsetzung von Intermediaten der allgemeinen Formel VI - die in an sich bekannter Weise aus den entsprechenden Alkoholen der Formel V hergestellt werden können - mit 1,2-Diaminocyclo-pentan bzw. -hexan zu Verbindungen der allgemeinen Formel VII und anschließender Abspaltung der Aminoschutzgruppe, erhalten werden, 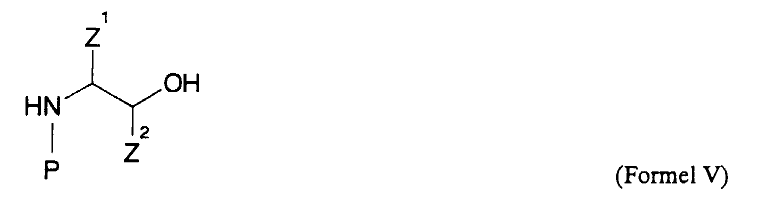 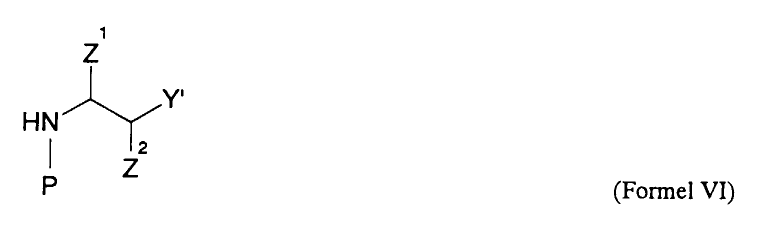 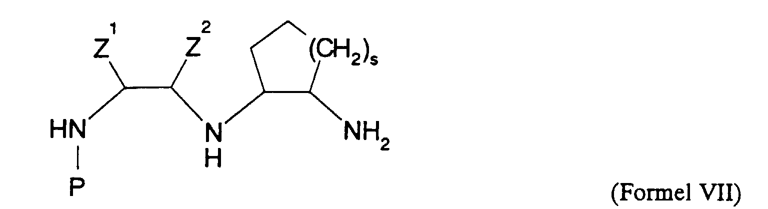 worin
Z¹, Z² und s die zuvor angegebene Bedeutung hat,
Y' eine Fluchtgruppe und
P für eine Aminoschutzgruppe steht.

Als Fluchtgruppe Y' seien beispielsweise Trifluormethylsulfonat, Brosylat und Nosylat, bevorzugt Tosylat und Mesylat genannt.
Als Aminoschutzgruppe P seien alle dem Fachmann bekannten Aminoschutzgruppen [siehe z.B. E. Wünsch, Methoden der Org. Chemie (Houben-Weyl), Band XV/1,4. Auflage 1974, S. 46 ff], bevorzugt eine Benzyloxycarbonylgruppe genannt.

Die Abspaltung der Schutzgruppen P erfolgt nach den dem Fachmann bekannten Verfahren, beispielsweise durch katalytische Hydrogenolyse, Behandlung mit Alkali oder mit anorganischen und/oder organischen Säuren wie beispielsweise Halogenwasserstoff in Eisessig.

Die Edukte der allgemeinen Formel V, in denen Z² für ein Wasserstoffatom steht, werden, soweit sie nicht käuflich erhältlich sind, hergestellt, indem man die entsprechenden Aminocarbonsäureester (siehe EP 0 405 704) in der dem Fachmann bekannten Weise zu Alkoholen reduziert (z.B. J. March, Advanced Organic Chemistry, Third Edition 1985, Verlag John Wiley & Sons, S. 1095 ff.).

Die Edukte der allgemeinen Formel V, in denen Z¹ für ein Wasserstoffatom steht, können beispielsweise nach W.C. Vincek, C.S. Aldrich et al., J. Med. Chem. 1981, 24:7-12, hergestellt werden.

Ein alternativer Syntheseweg für Triamine der allgemeinen Formel II, in denen Z² für ein Wasserstoffatom steht, benutzt die Boranreduktion von Amiden der allgemeinen Formel VIII 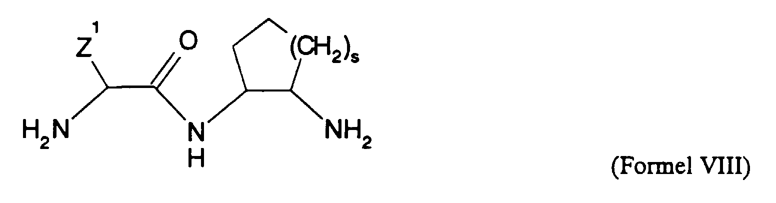 die durch Aminolyse von Aminosäureestern in literaturbekannter Weise erhalten werden können.

Die auf den verschiedenen Synthesewegen erhaltenen Komplexbildner der allgemeinen Formel I, können in die entsprechenden Metallkomplexe überführt werden. Auf eine vorherige Isolierung und Reinigung der Komplexbildner kann verzichtet werden.

Die Komplexierung der Metallionen der Elemente der genannten Ordnungszahlen erfolgt in der Weise, wie sie in der Patentschrift DE 34 01 052 offenbart worden ist, nämlich indem man das Metalloxid oder ein Metallsalz (beispielsweise das Nitrat, Acetat, Carbonat, Chlorid oder Sulfat) des gewünschten Elements in Wasser und/oder einem niederen Alkohol (wie Methanol, Ethanol oder Isopropanol) löst oder suspendiert und mit der Lösung oder Suspension der äquivalenten Menge des Komplexbildners der allgemeinen Formel I umsetzt, vorzugsweise bei Temperaturen zwischen 40 und 100°C, und anschließend gewünschtenfalls vorhandene acide Wasserstoffatome von Säuregruppen durch Kationen anorganischer und/oder organischer Basen, Aminosäuren oder Amino säureamide substituiert.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen, wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und anderen), niederen Ketonen (Aceton und andere), polaren Ethern (Tetrahydrofuran, Dioxan, 1,2-Dimethoxyethan und andere) auszufallen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten. Dies kann beispielsweise geschehen, indem man die komplexbildende Säure in wäßriger Suspension oder Lösung mit dem Oxid oder Salz des das Zentralion liefernden Elements umsetzt und gegebenenfalls teilweise mit der gewünschten Menge einer organischen Base versetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Komplexbildner (wie zum Beispiel Diethylentriaminpentaessigsäure) oder, falls erforderlich, Elektrolyte (wie zum Beispiel Natriumchlorid) oder, falls gewünscht, Calcium-, Magnesium oder Zinksalze organischer Säuren, wie z.B. von Ascorbinsäure, Gluconsäure, Diethylentriaminpentaessigsäure etc. Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) zum Beispiel Lecithine, Tween®, Myrj® und/oder Aromastoff(en) zur Geschmackskorrektur (zum Beispiel ätherischen Ölen) gemischt.

Darüber hinaus hat es sich als vorteilhaft erwiesen, dem Mittel einen Komplexbildnerüberschuß (der EP 0 270 483 entsprechend) z.B. in Form von Salzen mit gleichen oder Mischsalzen mit verschiedenen Kationen anorganischer und/oder organischer Basen, zuzusetzen.
Dadurch wird die Verträglichkeit des Kontrastmittels weiter gesteigert. Als besonders geeignete Kationen haben sich Calcium-, Magnesium-, Zink- und/oder Natrium erwiesen.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen. Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel werden je nach diagnostischer Fragestellung in einer Dosis von 0,1 µmol/kg bis 5 mmol/kg, vorzugsweise von 10 µmol bis 0,5 mmol/kg Körpergewicht des erfindungsgemäßen Komplexsalzes appliziert.
Bei der Anwendung der erfindungsgemäßen Mittel im Bereich der NMR-Diagnostik, werden diese im allgemeinen in Mengen von 1µmol-5mmol, vorzugsweise 5µmol-500µmol/kg Körpergewicht, dosiert. Details der Anwendung werden z.B. in HJ. Weinmann et al., Am. J. of Roentgenology, 142 (1984) 619 diskutiert.
Bei der Anwendung der erfindungsgemäßen Mittel im Bereich der Röntgendiagnostik werden diese im allgemeinen in Mengen von 100µmol-5mmol, vorzugsweise 250µmol-1mmol/kg Körpergewicht, dosiert. Details der Anwendung werden z.B. in Barke, Röntgenkontrastmittel, G. Thieme, Leipzig (1970) und P. Thurn, E. Bücheler, Einführung in die Röntgendiagnostik, G. Thieme, Stuttgart, New York (1977) diskutiert.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in einem wässrigen Medium löst und anschließend diese Lösung gegebenenfalls sterilisiert.

Bei einer intravenösen Injektion finden wäßrige Formulierungen der Konzentration 50 µmol/l bis 2 mol/l, vorzugsweise 100 mmol/l bis 1 mol/l, Verwendung. Bei oraler Anwendung werden vorzugsweise Lösungen der Konzentration 0,1 mmol/l bis 100 mmol/l verwendet, wobei gewünschtenfalls (ein) Aromastoff(e) zur Geschmackskorrektur (z.B. etherische Öle) beigefügt (wird) werden.
Bei rektaler Anwendung können ebenfalls Lösungen, in dem für die orale Anwendung genannten Konzentrationsbereich, verwendet werden. Darüber hinaus eignen sich vorzugsweise aber auch Suspensionen der erfindungsgemäßen Komplexe mit den in der Galenik üblichen Hilfsstoffen.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen gebundenen - an sich giftigen - Ionen innerhalb der Zeit, in der die neuen Kontrastmittel vollständig wieder ausgeschieden werden, praktisch nicht erfolgt.

Bei der Applikation der erfindungsgemäßen Mittel können diese auch zusammen mit einem geeigneten Träger wie z.B. Serum oder physiologischer Kochsalzlösung und/oder zusammen mit einem Protein wie z.B. Human Serum Albumin verabreicht werden. Die Dosierung ist dabei abhängig von der Art der zellulären Störung und den Eigenschaften des zur Anwendung kommenden Metallkomplexes.

Die erfindungsgemäßen Mittel erfüllen die vielfältigen Voraussetzungen für die Eignung als Kontrastmittel. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten. Als organspezifische Kontrastmittel sind sie von besonderem Wert für die Leber- und Gallendiagnostik. Sie sind darüberhinaus hervorragend als Perfusionsagentien geeignet.

Die erfindungsgemäßen Mittel können auch für die Strahlentherapie verwendet werden. So sind Komplexe des Gadoliniums aufgrund des großen Einfangquerschnitts für die Neutroneneinfangtherapie hervorragend geeignet. Ist das erfindungsgemäße Mittel zur Anwendung in der von R.L. Mills et al. [Nature Vol. 336 (1988) S. 787] vorgeschlagenen Variante der Strahlentherapie bestimmt, so muß sich das Zentralion von einem Mößbauer-Isotop wie z.B. ⁵⁷Fe oder ¹⁵¹Eu ableiten.

Die nachfolgenden Beispiele erläutern die Erfindung, ohne sie auf diese beschränken zu wollen.

### Beispiel 1

### a) N-Benzyloxycarbonyl-O-methyl-tyrosinmethylester

32,94 g (100 mmol) N-Benzyloxycarbonyl-tyrosinmethylester werden in 200 ml DMF mit 27,64 g (200 mmol) gemahlenem Kaliumcarbonat versetzt. Zu dieser Suspension werden 8,96 ml (110 mmol) Iodethan zugetropft und über Nacht bei Raumtemperatur gerührt. Die Lösung wird eingeengt, zwischen Essigester und Wasser verteilt und die organische Phase nach Trocknen (Na₂SO₄) mit Hexan versetzt. Die Titelverbindung kristallisiert aus.
Ausbeute: 32,88 g (92 %)
Fp: 50-56°C

| Analyse: | | | |
|---|---|---|---|
| ber.: | C 67,21 | H 6,49 | N 3,92 |
| gef.: | C 66,96 | H 6,57 | N 3,81 |

### b) N-Benzyloxycarbonyl-3-(4-ethoxybenzyl)-2-aminopropanol

Zu einer Lösung von 22,14 g (60,6 mmol) N-Benzyloxycarbonyl-O-ethyltyrosinmethylester (Beispiel la) in 150 ml Tetrahydrofuran werden bei Raumtemperatur 3,18 g (84,8 mmol) Natriumborhydrid zugegeben. Dazu werden unter Rühren 27 ml Methanol innerhalb 2 Stunden zugetropft. Anschließend wird das Tetrahydrofuran im Vakuum abdestilliert, der Rest in 100 ml Wasser aufgenommen und dreimal mit 70 ml Essigester extrahiert. Die vereinigte organische Phase wird mit Wasser gewaschen, mit Natriumsulfat getrocknet und aufkonzentriert. Es wird aus Essigester/Hexan umkristallisiert.
Ausbeute: 18,7 g (93,7 %)
Fp. 112-117°C

| Analyse: | | | |
|---|---|---|---|
| ber.: | C 69,28 | H 7,04 | N 4,25 |
| gef.: | C 69,02 | H 7,12 | N 4,13 |

### c) N-[2-Benzyloxycarbonylamino-3-(4-ethoxyphenyl)propyl]-cyclohexan-1,2-diamindihydrochlorid

Zu einer Lösung von 8,4 g (25,5 mmol) des im vorstehenden Beispiel beschriebenen Alkohols und 3,78 ml (27,3 mmol) Triethylamin in 33 ml Tetrahydrofuran werden bei 4°C langsam unter Rühren 2,09 ml (26,8 mmol) Methansulfonylchlorid zugetropft. Nach 2 Stunden wird der ausgefallene Niederschlag abfiltriert und das Filtrat zu 73.1 g (640 mmol) 1.2-Diamino-cyclohexan zugetropft. Die Lösung wird 4 Stunden bei 50°C gerührt und anschließend im Vakuum konzentriert. Der Rückstand wird mit Wasser aufgenommen, mit Ethylacetat extrahiert, mit Natriumsulfat getrocknet und am Rotationsverdampfer konzentriert. Der Rest wird in Methanol aufgenommen und bei 0°C mit konz. Salzsäure angesäuert. Nach Absaugen und Trocknen wird das Produkt in Form farbloser Kristalle (9,7 g; 76,3 %) als Dihydrochlorid erhalten.

| Analyse: | | | | |
|---|---|---|---|---|
| ber.: | C 60,24 | H 7,48 | Cl 14,22 | N 8,43 |
| gef.: | C 59,97 | H 7,33 | Cl 14,59 | N 8,21 |

### d) 8-(4-Ethoxybenzyl)-4,5-tetramethylen-3,6,9-tris(carboxymethyl)-3,6,9-triazaundecandisäure, Mono-Natriumsalz

7,48 g (15,0 mmol) des im vorstehenden Beispiel beschriebenen Triamins werden in 60 ml Methanol suspendiert, unter Stickstoff mit Palladium-Katalysator (10 % Pd auf Aktivkohle) versetzt und mit Wasserstoff begast. Nach Beendigung der Hydrierung wird vom Katalysator abgesaugt und das Filtrat eingeengt. Das resultierende Öl wird in 150 ml Tetrahydrofuran suspendiert, mit 10,37 g (75 mmol) Kaliumcarbonat und 7,5 ml Wasser versetzt und nach Zugabe von 21,94 g (112,5 mmol) Bromessigsäure-tert.-butylester 20 Stunden unter Rückfluß gerührt. Die organische Phase wird abgetrennt, zur Trockne eingedampft und das resultierende Öl an Kieselgel chromatographiert (Hexan/Ether/Triethylamin 70:30:5). Die eingedampften Fraktionen (7,94 g; 61 %) werden in Methanol gelöst und nach Zugabe von 9,15 ml 10 N Natronlauge 5 Stunden unter Rückfluß gerührt. Anschließend wird eingeengt und die wäßrige Lösung mit Amberlite® IR 120 (H⁺) Ionenaustauscher auf pH 3 eingestellt und die Lösung gefriergetrocknet. Man erhält das Mono-Natriumsalz des Komplexbildners.
Ausbeute: 5,57 g (57 %)
H₂O-Gehalt: 8,7 %

| Analyse (bezogen auf wasserfreie Substanz): | | | | |
|---|---|---|---|---|
| ber.: | C 53,73 | H 6,35 | N 6,96 | Na 3,81 |
| gef.: | C 53,30 | H 6,57 | N 6,62 | Na 4,09 |

### e) Gadoliniumkomplex der 8-(4-Ethoxybenzyl)-4,5-tetramethylen-3,6,9-tris(carboxylatomethyl)-3,6,9-triazaundecandisäure, Di-Natriumsalz

4,0 g (6,63 mmol) des in Beispiel 1d beschriebenen Komplexbildners werden in 40 ml Wasser gelöst und bei 80°C mit 1,20 g (3,31 mmol) Gd₂O₃ komplexiert. (Die Einstellung wird mit Xylenolorange-Indikatorlösung überprüft.) Nach Abkühlen gibt man 6,6 ml 1 N Natronlauge zu, die Lösung wird über Membranfilter (Sartorius Cellulose-nitrat 0,1 mm) filtriert und das Filtrat gefriergetrocknet.
Ausbeute: 5,55 g (quantitativ)
H₂O-Gehalt: 7,3 %
Fp: > 300°C

| Analyse (bezogen auf wasserfreie Substanz): | | | | | |
|---|---|---|---|---|---|
| ber.: | C 41,59 | H 4,39 | Gd 20,17 | N 5,39 | Na 5,90 |
| gef.: | C 41,17 | H 4,45 | Gd 19,79 | N 5,31 | Na 5,49 |

T₁-Relaxivität (H₂O): 5,9 ± 0,53 L/mmol·sec
T₁-Relaxivität (Plasma): 9,8 ± 0,84 L/mmol·sec

### Beispiel 2

### a) N-Benzyloxycarbonyl-O-methyl-tyrosinmethylester

32,94 g (100 mmol) N-Benzyloxycarbonyl-tyrosinmethylester werden in 200 ml DMF mit 27,64 g (200 mmol) gemahlenem Kaliumcarbonat versetzt. Zu dieser Suspension werden 15,6 g (110 mmol) Iodmethan zugetropft und über Nacht bei Raumtemperatur gerührt. Die Lösung wird eingeengt, zwischen Essigester und Wasser verteilt und die organische Phase nach Trocknen (Na₂SO₄) mit Hexan versetzt. Die Titelverbindung kristallisiert aus.
Ausbeute: 31,9 g (93 %)

| Analyse: | | | |
|---|---|---|---|
| ber.: | C 66,46 | H 6,16 | N 4,08 |
| gef.: | C 66,60 | H 6,23 | N 3,99 |

### b) Nₐ-Benzyloxycarbonyl-O-methyl-tyrosin-(2-aminocyclohexyl)-amid-hydrochlorid

24,0 g (70 mmol) N-Benzyloxycarbonyl-O-methyl-tyrosinmethylester werden in 50 ml Methanol gelöst und in ca. 2 Stunden in 420 ml (3,5 mol) trans-1,2-Diaminocyclohexan eingetropft. Die Lösung wird 24 Stunden bei Raumtemperatur gerührt und anschließend im Ölvakuum zur Trockne eingedampft. Der ölige Rückstand wird in Essigester aufgenommen und mehrmals zur Beseitigung von Diaminocyclohexan-Resten mit Wasser ausgeschüttelt. Die organische Phase wird getrocknet (Na₂SO₄) und mit 2N Chlorwasserstoff in Essigester versetzt. Der nach kurzer Zeit entstehende Niederschlag wird abfiltriert und bei 50°C im Vakuum getrocknet.
Ausbeute: 23,0 g (71 %)

| Analyse: | | | | |
|---|---|---|---|---|
| ber.: | C 62,40 | H 6,98 | Cl 7,67 | N 9,10 |
| gef.: | C 61,70 | H 7,05 | Cl 7,38 | N 9,25 |

### c) N-[2-Amino-3-(4-Methoxyphenyl)propyl]-cyclohexan-1,2-diamin-trihydrochlorid

18,5 g (40 mmol)Nₐ-Benzyloxycarbonyl-O-methyl-tyrosin-(2-aminocyclohexyl)-amidhydrochlorid werden in 200 ml Methanol suspendiert und unter Stickstoff mit Palladium auf Aktivkohle (10 % Pd) versetzt und wahlweise im Autoklaven oder unter Normaldruck mit Wasserstoff hydriert. Nach beendeter Reaktion (ca. 2-6 Stunden) wird vom Katalysator abgesaugt und das Filtrat eingeengt. Das erhaltene Öl wird in 320 ml 1 M Diboran/Tetrahydrofuran-Komplex-Lösung (320 mmol) suspendiert und 48 Stunden unter Rückfluß gerührt. Anschließend wird im Eisbad gekühlt und die Reaktion durch Zugabe von 15 ml Methanol beendet. Man läßt eine Stunde im Eisbad rühren und leitet dann Chlorwasserstoff ein, wobei das Trihydrochlorid des gewünschten Amins ausfällt. Der Niederschlag wird abgesaugt und über P₂O₅ getrocknet.
Ausbeute: 14,2 g (92 %)

| Analyse: | | | | |
|---|---|---|---|---|
| ber.: | C 49,68 | H 7,82 | Cl 27,50 | N 10,86 |
| gef.: | C 49,21 | H 7,70 | Cl 28,75 | N 10,20 |

### d) 8-(4-Methoxybenzyl)-4,5-tetramethylen-3,6,9-tris(carboxymethyl)-3,6,9-triazaundecandisäure, Mono-Natriumsalz

5,8 g (15 mmol) des im vorstehenden Beispiel beschriebenen Triamins werden in 150 ml Tetrahydrofuran suspendiert, mit 12,4 g (90 mmol) Kaliumcarbonat und 7,5 ml Wasser versetzt und nach Zugabe von 21,94 g (112,5 mmol) Bromessigsäure-tert.-butylester 20 Stunden unter Rückfluß gerührt. Die organische Phase wird abgetrennt, zur Trockne eingedampft und das resultierende Öl an Kieselgel chromatographiert (Hexan/ Ether/ Triethylamin 70:30:5). Die eingedampften Fraktionen (7,89 g; 62 %) werden in Methanol gelöst und nach Zugabe von 9,15 ml 10 N Natronlauge 5 Stunden unter Rückfluß gerührt. Anschließend wird eingeengt und die wäßrige Lösung mit Amberlite® IR 120 (H⁺) Ionenaustauscher auf pH 3 eingestellt und die Lösung gefriergetrocknet. Man erhält das Mono-Natriumsalz des Komplexbildners.
Ausbeute: 5,60 g (59 %)
H₂O-Gehalt: 6,9 %

| Analyse (bezogen auf wasserfreie Substanz): | | | | |
|---|---|---|---|---|
| ber.: | C 52,97 | H 6,15 | N 7,12 | Na 3,90 |
| gef.: | C 53,18 | H 6,01 | N 7,21 | Na 3,47 |

### e) Gadoliniumkomplex der 8-(4-Methoxybenzyl)-4,5-tetramethylen-3,6,9-tris(carboxylatomethyl)-3,6,9-triazaundecandisäure, Di-Natriumsalz

4,0 g (6,32 mmol) des in Beispiel 2d beschriebenen Komplexbildners werden in 40 ml Wasser gelöst und bei 80°C mt 1,15 g (3,16 mmol) Gd₂O₃ komplexiert. Nach Abkühlen gibt man 6,3 ml 1 N Natronlauge zu, die Lösung wird über Membranfilter (Sartorius Cellulose-nitrat 0,1 mm) filtriert und das Filtrat gefriergetrocknet.
Ausbeute: 5,3 g (quantitativ)
H₂O-Gehalt: 8,4 %

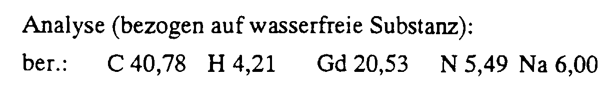

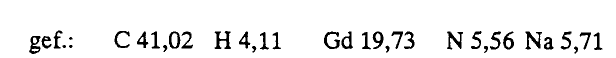

T₁-Relaxivität (H₂O): 6,2 ± 0,47 L/mmol·sec
T₁-Relaxivität (Plasma): 9,4 ± 0,56 L/mmol·sec

### Beispiel 3

### a) N-(2-Benzyloxycarbonylamino-3-phenylpropyl)-cyclopentyl-1,2-diamindihydrochlorid

Zu einer Lösung von 5,7 g (20 mmol) N-Benzyloxycarbonyl-phenylalaninol [Correa et al. Synth. Commun. 21, 1-9 (1991)] und 3,0 ml (21,7 mmol) Triethylamin in 30 ml Tetrahydrofuran werden bei 4°C langsam unter Rühren 1,64 ml (21 mmol) Methansulfonylchlorid zugetropft. Nach 2 Stunden wird der ausgefallene Niederschlag abfiltriert und das Filtrat zu 50,1 g (500 mmol) 1,2-Diaminocyclopentan [Jaeger und Blumendal, Z. anorg. Chem. 175, 161 (1928)] zugetropft. Die Lösung wird 4 Stunden bei 50°C gerührt und anschließend im Vakuum konzentriert. Der Rückstand wird mit Wasser aufgenommen, mit Ethylacetat extrahiert, mit Natriumsulfat getrocknet und am Rotationsverdampfer eingeengt. Das verbleibende Öl wird in Methanol aufgenommen und bei 0°C mit konz. Salzsäure angesäuert. Nach Absaugen und Trocknen wird das Produkt in amorpher Form erhalten.
Ausbeute: 7,1 g (81 %)

| Analyse: | | | | |
|---|---|---|---|---|
| ber.: | C 60,00 | H 7,09 | Cl 16,10 | N 9,54 |
| gef.: | C 59,43 | H 7,20 | Cl 16,62 | N 9,29 |

### b) 8-Benzyl-4,5-trimethylen-3,6,9-tris(carboxymethyl)-3,6,9-triazaundecandisäure, Mono-Natriumsalz

6,6 g (15 mmol) des im vorstehenden Beispiel beschriebenen Triamins werden in 60 ml Methanol suspendiert, unter Stickstoff mit Palladium-Katalysator (10 % Pd auf Aktivkohle) versetzt und mit Wasserstoff begast. Nach Beendigung der Hydrierung wird vom Katalysator abgesaugt und das Filtrat eingeengt. Das resultierende Öl wird in 150 ml Tetrahydrofuran suspendiert, mit 10,37 g (75 mmol) Kaliumcarbonat und 7,5 ml Wasser versetzt und nach Zugabe von 21,94 g (112,5 mmol) Bromessigsäure-tert.-butylester 20 Stunden unter Rückfluß gerührt. Die organische Phase wird abgetrennt, zur Trockne eingedampft und das resultierende Öl an Kieselgel chromatographiert (Hexan/Ether/ Triethylamin 70:30:5). Die eingedampften Fraktionen werden in Methanol gelöst und nach Zugabe von 9,15 ml 10 N Natronlauge 5 Stunden unter Rückfluß gerührt. Anschließend wird eingeengt und die wäßrige Lösung mit Amberlite® IR 120 (H⁺) Ionenaustauscher auf pH 3 eingestellt und die Lösung gefriergetrocknet. Man erhält das Mono-Natriumsalz des Komplexbildners.Ausbeute: 5,4 g (62 %) H₂O-Gehalt: 6,4 %

| Analyse (bezogen auf wasserfreie Substanz): | | | | |
|---|---|---|---|---|
| ber.: | C 52,84 | H 5,91 | N 7,70 | Na 4,21 |
| gef.: | C 53,17 | H 6,07 | N 7,62 | Na 4,03 |

### c) Gadoliniumkomplex der 8-Benzyl-4,5-trimethylen-3,6,9-tris(carboxylatomethyl)-3,6,9-triazaundecandisäure, Di-Natriumsalz

3,67 g (6,32 mmol) des in Beispiel 3b beschriebenen Komplexbildners wurden in 40 ml Wasser gelöst und bei 80°C mit 1,15 g (3,16 mmol) Gd₂O₃ komplexiert. Nach Abkühlen gibt man 6,3 ml 1 N Natronlauge zu, die Lösung wird über Membranfilter (Sartorius Cellulose-nitrat 0,1 mm) filtriert und das Filtrat gefriergetrocknet.
Ausbeute: 4,97 g (quantitativ)
H₂O-Gehalt: 9,2 %

| Analyse (bezogen auf wasserfreie Substanz): | | | | | |
|---|---|---|---|---|---|
| ber.: | C 39,94 | H 3,91 | Gd 21,79 | N 5,82 | Na 6,37 |
| gef.: | C 40,47 | H 3,84 | Gd 21,31 | N 5,62 | Na 6,29 |

T₁-Relaxivität (H₂O): 5,1 ± 0,39 L/mmol·sec
T₁-Relaxivität (Plasma): 8,8 ± 0,72 L/mmol·sec

### Beispiel 4

### a) N-(2-Benzyloxycarbonylamino-4-methylpentyl)-cyclohexyl-1,2-diamindihydrochlorid

Zu einer Lösung von 5,0 g (20 mmol) N-Benzyloxycarbonyl-leucinol [Correa et al. Synth. Commun. 21, 1-9 (1991)] und 3,0 ml (21,7 mmol) Triethylamin in 30 ml Tetrahydrofuran werden bei 4°C langsam unter Rühren 1,64 ml (21 mmol) Methansulfonylchlorid zugetropft. Nach 2 Stunden wird der ausgefallene Niederschlag abfiltriert und das Filtrat zu 120 ml (1 mol) trans-1,2-Diaminocyclohexan zugetropft. Die Lösung wird 4 Stunden bei 50°C gerührt und anschließend im Vakuum konzentriert. Der Rückstand wird mit Wasser aufgenommen, mit Ethylacetat extrahiert, mit Natriumsulfat getrocknet und am Rotationsverdampfer eingeengt. Das verbleibende Öl wird in Methanol aufgenommen und bei 0°C mit konz. Salzsäure angesäuert. Nach Absaugen und Trocknen wird das Produkt in amorpher Form erhalten.
Ausbeute: 7,1 g (84 %)

| Analyse: | | | | |
|---|---|---|---|---|
| ber.: | C 57,14 | H 8,39 | Cl 16,87 | N 9,99 |
| gef.: | C 57,43 | H 8,20 | Cl 16,22 | N 9,69 |

### b) 8-(2-Methylpropyl)-4,5-tetramethylen-3,6,9-tris(carboxymethyl)-3,6,9-triazaundecandisäure, Mono-Natriumsalz

6,3 g (15 mmol) des im vorstehenden Beispiel beschriebenen Triamins werden in 60 ml Methanol suspendiert, unter Stickstoff mit Palladium-Katalysator (10 % Pd auf Aktivkohle) versetzt und mit Wasserstoff begast. Nach Beendigung der Hydrierung wird vom Katalysator abgesaugt und das Filtrat eingeengt. Das resultierende Öl wird in 150 ml Tetrahydrofuran suspendiert, mit 10,37 g (75 mmol) Kaliumcarbonat und 7,5 ml Wasser versetzt und nach Zugabe von 21,94 g (112,5 mmol) Bromessigsäure-tert.-butylester 20 Stunden unter Rückfluß gerührt. Die organische Phase wird abgetrennt, zur Trockne eingedampft und das resultierende Öl an Kieselgel chromatographiert (Hexan/Ether/ Triethylamin 70:30:5). Die eingedampften Fraktionen werden in Methanol gelöst und nach Zugabe von 9,15 ml 10 N Natronlauge 5 Stunden unter Rückfluß gerührt. Anschließend wird eingeengt und die wäßrige Lösung mit Amberlite® IR 120 (H⁺) Ionenaustauscher auf pH 3 eingestellt und die Lösung gefriergetrocknet. Man erhält das Mono-Natriumsalz des Komplexbildners.
Ausbeute: 5,1 g (61 %)
H₂O-Gehalt: 7,2 %

| Analyse (bezogen auf wasserfreie Substanz): | | | | |
|---|---|---|---|---|
| ber.: | C 50,28 | H 6,90 | N 8,00 | Na 4,37 |
| gef.: | C 49,95 | H 7,07 | N 7,66 | Na 3,96 |

### c) Gadoliniumkomplex der 8-(2-Methylpropyl)-4,5-tetramethylen-3,6,9-tris(carboxylatomethyl)-3,6,9-triaazaundecandisäure, Di-Natriumsalz

3,73g (6,63 mmol) des in Beispiel 4b beschriebenen Komplexbildners werden in 40 ml Wasser gelöst und bei 80°C mit 1,20 g (3,31 mmol) Gd₂O₃ komplexiert. (Die Einstellung wird mit Xylenolorange-Indikatorlösung überprüft.) Nach Abkühlen gibt man 6,6 ml 1 N Natronlauge zu, die Lösung wird über Membranfilter (Sartorius Cellulose-nitrat 0,1 mm) filtriert und das Filtrat gefriergetrocknet.
Ausbeute: 5,02 g (quantitativ)
H₂O-Gehalt: 82 %

| Analyse (bezogen auf wasserfreie Substanz): | | | | | |
|---|---|---|---|---|---|
| ber.: | C 37,66 | H 4,60 | Gd 22,41 | N 5,99 | Na 6,55 |
| gef.: | C 37,37 | H 4,45 | Gd 21,79 | N 5,61 | Na 6,49 |

T₁-Relaxivität (H₂O): 4,9 ± 0,23 L/mmol·sec
T₁-Relaxivität (Plasma): 8,1 ± 0,54 L/mmol·sec

### Beispiel 5

### Gadoliniumkomplex der 8-(4-Ethoxycyclohexylmethyl)-4,5-tetramethylen-3,6,9-tris(carboxylatomethyl)-3,6,9-triazaundecandisäure, Di-Natriumsalz

5 g (6,41 mmol) der Titelverbindung aus Beispiel le werden in 50 ml Wasser gelöst und 3 g Rhodium-Katalysator (Rh auf Al₂O₃) zugesetzt. Die Lösung wird in einem Autoklaven 48 Stunden unter 10 bar Wasserstoffdruck bei 40°C hydriert. Der Katalysator wird abfiltriert und das Filtrat gefriergetrocknet.
Ausbeute: 5,38 g (98,5 %)
H₂O-Gehalt: 7,8 %

| Analyse (bezogen auf wasserfreie Substanz): | | | | | |
|---|---|---|---|---|---|
| ber.: | C 41,27 | H 5,13 | Gd 20,01 | N 5,35 | Na 5,85 |
| gef.: | C 40,95 | H 5,29 | Gd 19,83 | N 5,18 | Na 5,41 |

T₁-Relaxivität (H₂O): 6,2 ± 0,61 L/mmol·sec
T₁-Relaxivität (Plasma): 9,7 ± 0,43 L/mmol·sec

## Patentansprüche

1. Komplexverbindungen bestehend aus mindestens einem Metallion eines Elementes der Ordnungszahlen 21-29, 42, 44 oder 58-83 und einem Komplexbildner der allgemeinen Formel I 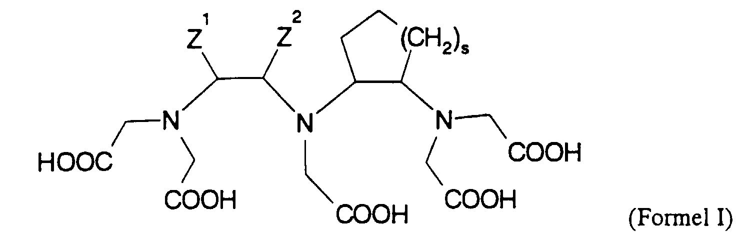 worin
Z¹ und Z² unabhängig voneinander für ein Wasserstoffatom, den Rest -(CH₂)ₘ-(C₆H₄)_{q}-(O)ₖ-(CH₂)ₙ-(C₆H₄)ₗ-(O)ᵣ-R oder den Rest -(CH₂)ₘ-(C₆H₁₀)_{q}-(O)ₖ-(CH₂)ₙ-(C₆H₁₀)₁-(O)ᵣ-R stehen,
worin
m und n die Ziffern 0-5
q, k, l und r die Ziffern 0 oder 1 und
s die Ziffern 1 oder 2 bedeuten,
R ein Wasserstoffatom, einen gegebenenfalls OR¹-substituierten geradkettigen oder verzweigten C₁-C₆-Alkylrest oder eine CH₂-COOR¹-Gruppe,
worin R¹ ein Wasserstoffatom, ein C₁-C₆-Alkylrest oder eine Benzylgruppe bedeutet,
unter den Maßgaben, daß jeweils einer der Substituenten Z¹ oder Z² für ein Wasserstoffatom steht und der andere nicht für Wasserstoff steht und daß eine direkte Sauerstoff-Sauerstoff Bindung nicht zugelassen ist,
wobei freie Carbonsäuregruppen, das heißt Carbonsäuregruppen die nicht zur Komplexierung des Metallions der genannten Ordnungszahlen benötigt werden, gewünschtenfalls teilweise oder vollständig als Salz einer Aminosäure oder eines Aminosäureamids oder als Salz einer anorganischen oder organischen Base vorliegen.

2. Komplexverbindungen nach Anspruch 1, dadurch gekennzeichnet, daß Z² für ein Wasserstoffatom und Z¹ für einen Rest -(CH₂)ₘ-(C₆H₄)_{q}-(O)ₖ-(CH₂)ₙ-(C₆H₄)ₗ-(O)ᵣ-R oder den Rest -(CH₂)ₘ-(C₆H₁₀)_{q}-(O)ₖ-(CH₂)ₙ-(C₆H₄)ₗ-(O)ᵣ-R steht.

3. Komplexverbindungen nach Anspruch 1, dadurch gekennzeichnet, daß Z² für ein Wasserstoffatom und Z¹ für die Reste iso-Butyl-, n-Pentyl-, -CH₂-C₆H₄-OH, -CH₂-C₆H₄-OCH₃, -CH₂-C₆H₄-O-C₂H₅, -CH₂-C₆H₄-O-C₄H₉, -CH₂-C₆H₄-O-CH₂-C₆H₅, -CH₂-C₆H₅, -CH₂-C₆H₁₀-O-C₂H₅, -CH₂-C₆H₄-O-CH₂-C₆H₄-OCH₃, -CH₂-O-CH₂-C₆H₅, -CH₂-C₆H₄-O-CH₂-COOH steht.

4. Pharmazeutische Mittel enthaltend mindestens eine physiologisch verträgliche Komplexverbindung nach Anspruch 1-3, gegebenenfalls mit den in der Galenik üblichen Zusätzen.

5. Verwendung von mindestens einer physiologisch verträglichen Komplexverbindung gemäß Anspruch 1-3 für die Herstellung von Mitteln für die Röntgen- und NMR-Diagnostik.

6. Verwendung von mindestens einer physiologisch verträglichen Komplexverbindung gemäß Anspruch 1-3 für die Herstellung von Mitteln für die Röntgen- und NMR-Diagnostik des hepatobiliären Systems.

7. Verwendung von mindestens einer physiologisch verträglichen Komplexverbindung gemäß Anspruch 1-3 für die Herstellung von Mitteln für die Strahlentherapie.

8. Verfahren zur Herstellung von Komplexverbindungen bestehend aus mindestens einem Metallion eines Elementes der Ordnungszahlen 21-29, 42, 44 oder 58-83 und einem Komplexbildner der allgemeinen Formel I 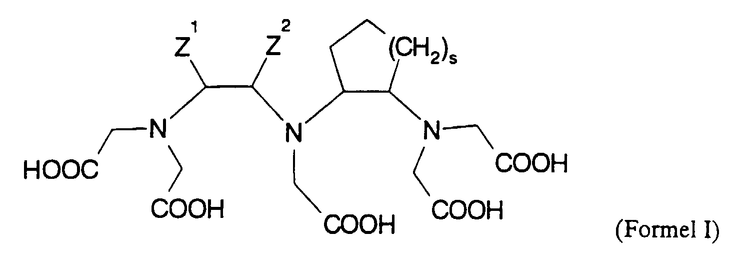 worin
Z¹ und Z² unabhängig voneinander für ein Wasserstoffatom, den Rest -(CH₂)ₘ-(C₆H₄)_{q}-(O)ₖ-(CH₂)ₙ-(C₆H₄)ₗ-(O)ᵣ-R oder den Rest -(CH₂)ₘ-(C₆H₁₀)_{q}-(O)ₖ-(CH₂)ₙ-(C₆H₄)ₗ-(O)ᵣ-R stehen,
worin
m und n die Ziffern 0-5
q, k, l und r die Ziffern 0 oder 1 und
s die Ziffern 1 oder 2 bedeuten,
R ein Wasserstoffatom, einen gegebenenfalls OR¹-substituierten geradkettigen oder verzweigten C₁-C₆-Alkylrest oder eine CH₂-COOR¹-Gruppe,
worin R¹ ein Wasserstoffatom, ein C₁-C₆-Alkylrest oder eine Benzylgruppe bedeutet,
unter den Maßgaben, daß jeweils einer der Substituenten Z¹ oder Z² für ein Wasserstoffatom steht und der andere nicht für Wasserstoff steht und daß eine direkte Sauerstoff-Sauerstoff Bindung nicht zugelassen ist,
wobei freie Carbonsäuregruppen, das heißt Carbonsäuregruppen die nicht zur Komplexierung des Metallions der genannten Ordnungszahlen benötigt werden, gewünschtenfalls teilweise oder vollständig als Salz einer Aminosäure oder eines Aminosäureamids oder als Salz einer anorganischen oder organischen Base vorliegen, dadurch gekennzeichnet, daß man
a) ein Triamin der allgemeinen Formel II 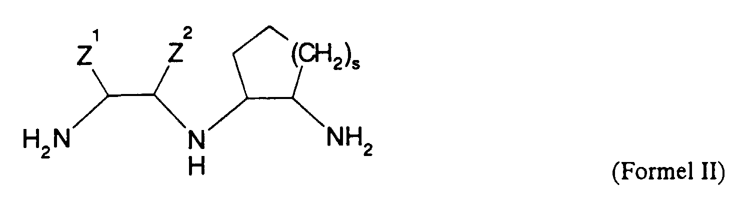 worin Z¹, Z² und s die angegebenen Bedeutungen haben
gegebenenfalls in Gegenwart einer Base, mit einer Verbindung der allgemeinen Formel III
Y-CH₂-COOR² (Formel III),
worin
Y eine Fluchtgruppe und
R² eine Säureschutzgruppe oder ein Wasserstoffatom bedeuten, in einem polaren Lösungsmittel bei -10°C bis 170°C innerhalb von 1-100 Stunden umsetzt, gewünschtenfalls Schutzgruppen abspaltet oder
b) eine Verbindung der allgemeinen Formel IV 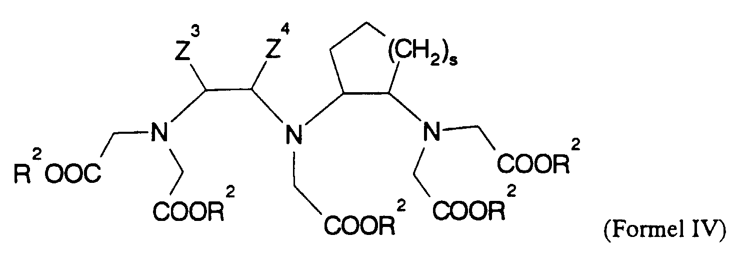 worin
R² und s die angegebenen Bedeutungen haben und
Z³ und Z⁴ jeweils für ein Wasserstoffatom oder den Rest -(CH₂)ₘ-(C₆H ₄)_{q}-OH stehen,
mit der Maßgabe, daß einer Substituenten Z³ und Z⁴ für ein Wasserstoffatom und der andere für den angegebenen Rest steht,
in eine Verbindung mit dem für Z¹ und Z² angegebenen Rest umwandelt und die Säureschutzgruppe R² abspaltet
und anschließend den gemäß der Varianten a) oder b) erhaltenen Komplexbildner der allgemeinen Formel I mit mindestens einem Metalloxid oder Metallsalz eines Elementes der Ordnungszahlen 21-29, 42, 44 oder 58-83 umsetzt und abschließend gewünschtenfalls acide Wasserstoffatome, der nicht zur Komplexierung benötigten freien Carbonsäuregruppen, durch Kationen anorganischer und/oder organischer Basen, Aminosäuren oder Aminosäureamiden substituiert.

## Claims

1. Complex compounds consisting of at least one metal ion of an element having an atomic number of from 21 to 29, 42, 44 or from 58 to 63 and a complex former of the general formula I 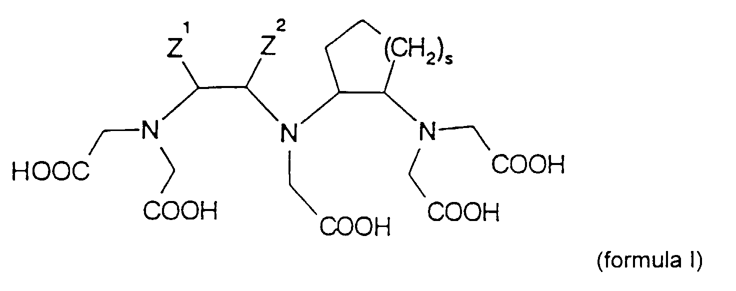 wherein Z¹ and Z² each independently of the other represent a hydrogen atom, the radical -(CH₂)ₘ-(C₆H₄)_{q}-(O)ₖ-(CH₂)ₙ-(C₆H₄)ₗ-(O)ᵣ-R or the radical -(CH₂)ₘ-(C₆H₁₀)_{q}-(O)ₖ-(CH₂)ₙ-(C₆H₁₀)ₗ-(O)ᵣ-R,
wherein
m and n represent the numbers 0 to 5,
q, k, l and r represent the numbers 0 or 1 and
s represents the numbers 1 or 2,
R represents a hydrogen atom, an unsubstituted or OR¹-substituted straight-chained or branched C₁-C₆alkyl radical or a CH₂-COOR¹ group,
wherein R¹ represents a hydrogen atom, a C₁-C₆alkyl radical or a benzyl group,
with the provisos that in each case one of the substituents Z¹ or Z² represents a hydrogen atom and the other does not represent hydrogen and that a direct oxygen-oxygen bond is not permitted,
one or more free carboxylic acid groups, that is to say carboxylic acid groups that are not required for complexing the metal ion of the mentioned atomic numbers, being if desired in the form of a salt of an amino acid or of an amino acid amide or in the form of a salt of an inorganic or organic base.

2. Complex compounds according to claim 1, characterised in that Z² represents a hydrogen atom and Z¹ represents a radical -(CH₂)ₘ-(C₆H₄)_{q}-(O)ₖ-(CH₂)ₙ-(C₆H₄)ₗ-(O)ᵣ-R or the radical -(CH₂)ₘ-(C₆H₁₀)_{q}-(O)ₖ-(CH₂)ₙ-(C₆H₁₀)ₗ-(O)ᵣ-R.

3. Complex compounds according to claim 1, characterised in that Z² represents a hydrogen atom and Z¹ represents the radicals isobutyl, n-pentyl, -CH₂-C₆H₄-OH, -CH₂-C₆H₄-OCH₃, -CH₂-C₆H₄-O-C₂H₅, -CH₂-C₆H₄-O-C₄H₉, -CH₂-C₆H₄-O-CH₂-C₆H₅, -CH₂-C₆H₅, -CH₂-C₆H₁₀-O-C₂H₅, -CH₂-C₆H₄-O-CH₂-C₆H₄-OCH₃, -CH₂-O-CH₂-C₆H₅ or -CH₂-C₆H₄-O-CH₂-COOH.

4. Pharmaceutical compositions comprising at least one physiologically tolerable complex compound according to claims 1 to 3, optionally together with the additives customary in galenical pharmacy.

5. Use of at least one physiologically tolerable complex compound according to claims 1 to 3 for the preparation of compositions for X-ray and NMR diagnostics.

6. Use of at least one physiologically tolerable complex compound according to claims 1 to 3 for the preparation of compositions for X-ray and NMR diagnostics of the hepatobiliary system.

7. Use of at least one physiologically tolerable complex compound according to claims 1 to 3 for the preparation of compositions for radiotherapy.

8. Process for the preparation of complex compounds consisting of at least one metal ion of an element having an atomic number of from 21 to 29, 42, 44 or from 58 to 63 and a complex former of the general formula I 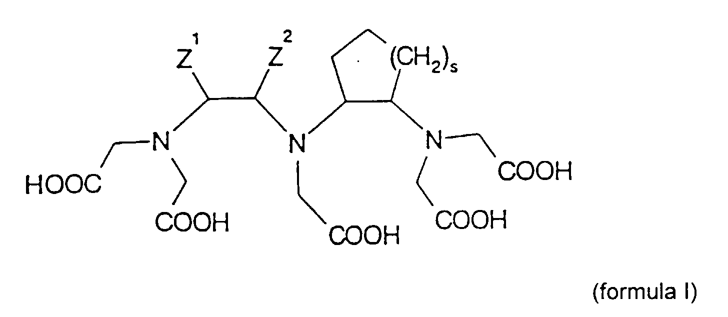 wherein Z¹ and Z² each independently of the other represent a hydrogen atom, the radical -(CH₂)ₘ-(C₆H₄)_{q}-(O)ₖ-(CH₂)ₙ-(C₆H₄)ₗ-(O)ᵣ-R or the radical -(CH₂)ₘ-(C₆H₁₀)_{q}-(O)ₖ-(CH₂)ₙ-(C₆H₄)ₗ-(O)ᵣ-R,
wherein
m and n represent the numbers 0 to 5,
q, k, l and r represent the numbers 0 or 1 and
s represents the numbers 1 or 2,
R represents a hydrogen atom, an unsubstituted or OR¹-substituted straight-chained or branched C₁-C₆alkyl radical or a CH₂-COOR¹ group,
wherein R¹ represents a hydrogen atom, a C₁-C₆alkyl radical or a benzyl group,
with the provisos that in each case one of the substituents Z¹ or Z² represents a hydrogen atom and the other does not represent hydrogen and that a direct oxygen-oxygen bond is not permitted,
one or more free carboxylic acid groups, that is to say carboxylic acid groups that are not required for complexing the metal ion of the mentiond atomic numbers, being if desired in the form of a salt of an amino acid or of an amino acid amide or in the form of a salt of an inorganic or organic base,
characterised in that
a) a triamine of the general formula II 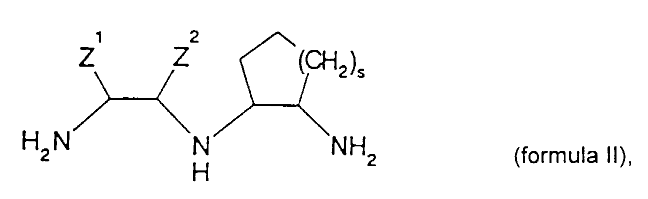 wherein Z¹, Z² and s have the meanings mentioned, is reacted, optionally in the presence of a base, with a compound of the general formula III
Y-CH₂-COOR² (formula III)
wherein
Y is a leaving group and
R² is an acid-protecting group or a hydrogen atom,
in a polar solvent at from -10°C to 170°C in the course of from 1 to 100 hours, if desired protecting groups are removed or
b) a compound of the general formula IV 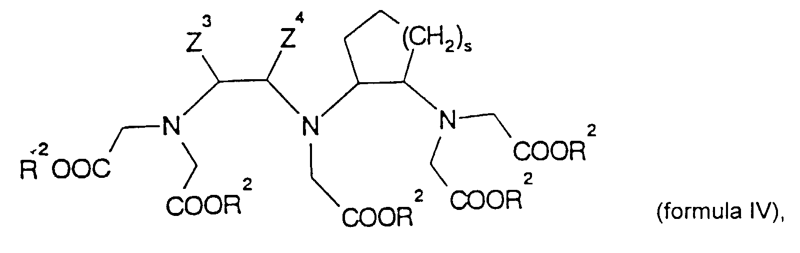 wherein
R² and s have the meanings mentioned and
Z³ and Z⁴ each represent a hydrogen atom or the radical -(CH₂)ₘ-(C₆H₄)_{q}-OH, with the proviso that one substituent Z³ or Z⁴ represents a hydrogen atom and the other the radical indicated,
is converted into a compound containing the radical indicated for Z¹ and Z² and the acid-protecting group R² is removed,
and then the complex former of the general formula I obtained in accordance with variant a) or b) is reacted with at least one metal oxide or metal salt of an element having an atomic number of from 21 to 29, 42, 44 or 58 to 83 and finally, if desired, acid hydrogen atoms of the free carboxylic acid groups not required for the complexing are replaced by cations of inorganic and/or organic bases, amino acids or amino acid amides.

## Revendications

1. Composés complexes constitués d'au moins un ion métallique d'un élément ayant un nombre atomique de 21 à 29, 42, 44 ou de 58 à 83 et d'un agent complexant de formule générale I 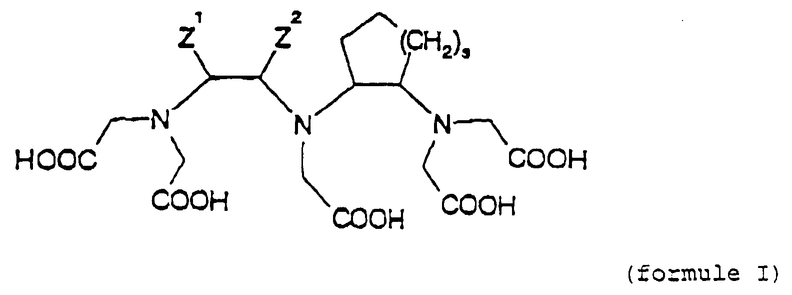 dans laquelle
Z¹ et Z² indépendamment l'un de l'autre représentent un atome d'hydrogène, le radical
-(CH₂)ₘ-(C₆H₄)_{q}-(O)ₖ-(CH₂)ₙ-(C₆H₄)ₗ-(O)ᵣ-R ou le radical - (CH₂)ₘ-(C₆H₁₀)_{q}-(O)ₖ-(CH₂)ₙ-(C₆H₁₀)ₗ-(O)ᵣ-R,
où
m et n vont de 0 à 5,
q, k, l et r valent 0 ou 1 et
s vaut 1 ou 2,
R représente un atome d'hydrogène, un radical alkyle en C₁-C₆ linéaire ou ramifié éventuellement substitué par OR¹ ou un groupe CH₂-COOR¹,
où R¹ représente un atome d'hydrogène, un radical alkyle en C₁-C₆ ou un groupe benzyle,
à la condition que chaque fois l'un des substituants Z¹ ou Z² représente un atome d'hydrogène et l'autre ne représente pas un atome d'hydrogène et qu'une liaison directe oxygène-oxygène ne soit pas permise,
les groupes acides carboxyliques libres, c'est-à-dire, les groupes acides carboxyliques n'étant pas nécessaires à la complexation de l'ion métallique ayant les nombres atomiques précités, étant présents partiellement ou entièrement sous forme d'un sel d'un aminoacide ou d'un amide d'aminoacide ou sous forme de sel d'une base organique ou inorganique.

2. Composés complexes selon la revendication 1, caractérisés en ce que Z² représente un atome d'hydrogène et Z¹ un radical -(CH₂)ₘ-(C₆H₄)_{q}-(O)ₖ-(CH₂)ₙ-(C₆H₄)ₗ-(O)ᵣ-R ou le radical -(CH₂)ₘ-(C₆H₁₀)_{q}-(O)ₖ-(CH₂)ₙ-(C₆H₄)ₗ-(O)ᵣ-R.

3. Composés complexes selon la revendication 1, caractérisés en ce que Z² représente un atome d'hydrogène et Z¹ les radicaux iso-butyle, n-pentyle, -CH₂-C₆H₄-OH, -CH₂-C₆H₄-OCH₃, -CH₂-C₆H₄-O-C₂H₅, -CH₂-C₆H₄-O-C₆H₉, -CH₂-C₆H₄-O-CH₂-C₆H₅, -CH₂-C₆H₅, -CH₂-C₆H₁₀-O-C₂H₅, -CH₂-C₆H₄-O-CH₂-C₆H₄-O-CH₃, -CH₂-O-CH₂-C₆H₅, -CH₂-C₆H₄-O-CH₂-COOH.

4. Agents pharmaceutiques contenant au moins un composé complexe physiologiquement compatible selon la revendication 1 à 3, éventuellement avec des adjuvants usuels en galénique.

5. Utilisation d'au moins un composé complexe physiologiquement compatible selon la revendication 1 à 3 pour la préparation d'agents pour le diagnostic aux rayons X ou par la RMN.

6. Utilisation d'au moins un composé complexe physiologiquement compatible selon la revendication 1 à 3 pour la préparation d'agents pour le diagnostic du système hépato-biliaire aux rayons X et par la RMN.

7. Utilisation d'au moins un des composés complexes physiologiquement compatibles selon la revendication 1 à 3 pour la préparation d'agents pour la thérapie par rayonnement.

8. Procédé pour la préparation d'au moins un composé complexe physiologiquement compatible composé d'un ions métallique d'un élément ayant un nombre atomique de 21 à 29, 42, 44 ou de 58 à 83 et d'un agent complexant de formule générale I 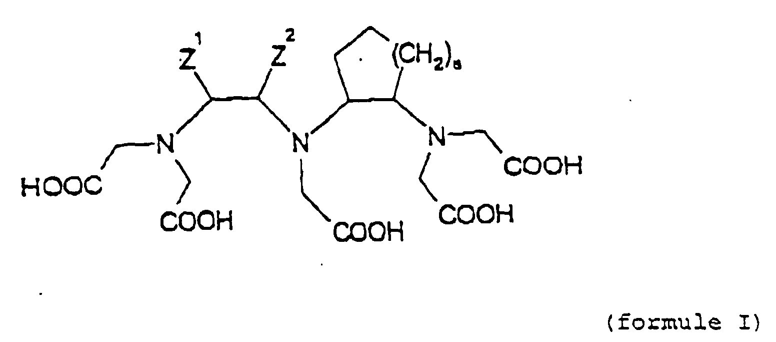 dans laquelle
Z¹ et Z², indépendamment l'un de l'autre, représentent un atome d'hydrogène, le radical
-(CH₂)ₘ-(C₆H₄)_{q}-(O)ₖ-(CH₂)ₙ-(C₆H₄)ₗ-(O)ᵣ-R ou le radical -(CH₂)ₘ-(C₆H₁₀)_{q}-(O)ₖ-(CH₂)ₙ-(C₆H₄)ₗ-(O)ᵣ-R,
où
m et n vont de 0 à 5,
q, k, l et r valent 0 ou 1 et
s vaut 1 ou 2,
R représente l'hydrogène, un radical alkyle en C₁-C₆ linéaire ou ramifié éventuellement substitué par OR¹ ou un groupe CH₂-COOR¹,
où R¹ représente un atome d'hydrogène, un radical alkyle en C₁-C₆ ou un groupe benzyle,
à la condition que chaque fois l'un des substituants Z¹ ou Z² représente l'hydrogène et l'autre ne soit pas un atome d'hydrogène et qu'une liaison directe oxygène-oxygène ne soit pas permise,
les groupes acides carboxyliques libres, c'est-à-dire, les groupes acides carboxyliques n'étant pas nécessaires à la complexation de l'ion métallique ayant les nombres atomiques précités, étant présents, si on le souhaite, partiellement ou entièrement sous forme d'un sel d'un aminoacide ou d'un amide d'aminoacide ou sous forme de sel d'une base organique ou inorganique, caractérisé en ce que
a) on fait réagir une triamine de formule générale II 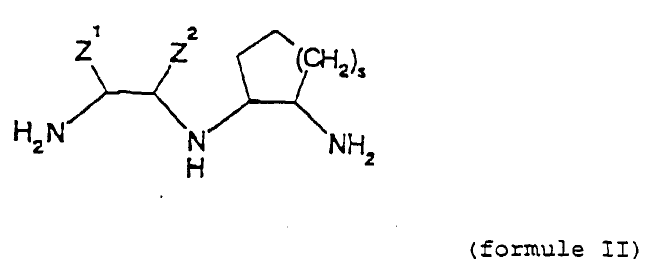 dans laquelle Z¹ et Z² et s ont les significations données, éventuellement en présence d'une base, avec un composé de formule générale III
Y-CH₂-COOR² (formule III)
dans laquelle
Y est un groupe éliminable et
R² est un groupe protecteur d'acide ou un atome d'hydrogène, dans un solvant polaire, à une température de -10°C à 170°C, en l'espace de 1 à 100 heures,
si on le souhaite, on sépare les groupes éliminables, ou
b) on transforme un composé de formule générale IV 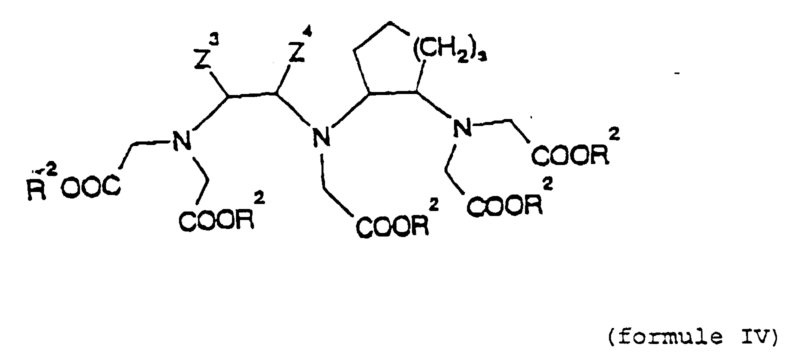 où
R² et s ont les significations données et
Z³ et Z⁴ chacun représente un atome d'hydrogène ou le radical -(CH₂)ₘ-(C₆H₄)_{q}-OH
à la condition que l'un des substituants Z³ et Z⁴ soit un atome d'hydrogène et l'autre le radical indiqué,
en un composé avec le radical indiqué pour Z¹ et Z² et on sépare le groupe protecteur d'acide R²
et ensuite, on fait réagir l'agent complexant de formule générale I obtenu selon les variantes a) ou b) avec au moins un oxyde métallique ou sel métallique d'un élément ayant un nombre atomique de 21 à 29, 42, 44, ou de 58 à 83 et finalement, si on le souhaite, on substitue les atomes d'hydrogène acides des groupes acides carboxyliques libres n'étant pas nécessaires à la complexation, par des cations de bases inorganiques et/ou organiques, par des aminoacides ou amides d'aminoacides.
